# EUROPÄISCHE PATENTSCHRIFT

(11) **EP 1 864 979 B1**
(45) Veröffentlichungstag und Bekanntmachung des Hinweises auf die Patenterteilung: **07.09.2011**
(21) Anmeldenummer: 07017340.6
(22) Anmeldetag: 07.04.2005
(51) Int. Cl.: C07D 401/06, C07D 401/14, C07D 409/14, C07D 417/06, A61K 31/435, A61K 31/41, A61P 7/00

(54) **Imidazol-Derivate als Tafia-Inhibitoren**
Imidazole-derivatives as TAFAIa-inhibitors
Dérivés d'imidazole comme inhibiteurs de TAFALA

(30) Priorität: 22.04.2004 DE 102004020186
(43) Veröffentlichungstag der Anmeldung: 12.12.2007
(62) Teilanmeldung aus: 05731221.7
(73) Patentinhaber: Sanofi-Aventis Deutschland GmbH, 65929 Frankfurt am Main (DE)
(72) Erfinder: Kallus, Christopher, 65926 Frankfurt am Main (DE); Heitsch, Holger, 65926 Frankfurt am Main (DE); Lindenschmidt, Andreas, 65926 Frankfurt am Main (DE); Grueneberg, Sven, 65926 Frankfurt am Main (DE); Szillat, Hauke, 65926 Frankfurt am Main (DE)

(56) Entgegenhaltungen:
- WO-A-03/013526
- BARROW ET AL.: "Synthesis and Evaluation of Imidazole Acetic Acid Inhibitors of Activated Thrombin-Activatable Fibrinolysis Inhibitor as Novel Antithrombotics" J. MED. CHEM., Bd. 46, 2003, Seiten 5294-5297, XP002338234

## Beschreibung

Die Erfindung betrifft neue Verbindungen der Formel Ia, die das Enzym TAFIa (aktivierter Thrombin-aktivierbarer Fibrinolyse Inhibitor) inhibieren, Verfahren zu ihrer Herstellung und Verwendung derselben als Arzneimittel.

Das Enzym TAFIa entsteht beispielsweise durch Thrombinaktivierung aus dem Thrombin-aktivierbaren-Fibrinolyse-Inhibitor-Zymogen (TAFI). Das Enzym TAFI wird auch als Plasma Procarboxypeptidase B, Procarboxypeptidase U oder als Procarboxypeptidase R bezeichnet und ist ein Carboxypeptidase B ähnliches Proenzym (L. Bajzar, Arterioscler. Thromb. Vasc. Biol. 2000, Seiten 2511 - 2518).

Während einer Gerinnselbildung wird Thrombin als das Endprodukt der Koagulationskaskade generiert und induziert die Konversion von löslichem Plasmafibrinogen zu einer unlöslichen Fibrinmatrix. Gleichzeitig aktiviert Thrombin den endogenen Fibrinolyse-Inhibitor TAFI. Aktiviertes TAFI (TAFIa) entsteht also während der Thrombusbildung und der Lyse aus dem Zymogen TAFI unter der Einwirkung von Thrombin, Thrombomodulin im Komplex mit Thrombin verstärkt diesen Effekt etwa 1250 fach. TAFIa spaltet basische Aminosäuren am carboxy-Ende der Fibrinfragmente. Der Verlust der carboxy-terminalen Lysine als Bindestellen für Plasminogen führt dann zu einer Inhibition der Fibrinolyse. Effektive Inhibitoren von TAFIa verhindern den Verlust dieser hoch affinen Lysin-Bindungsstellen für Plasminogen und unterstützen auf diese Weise die endogene Fibrinolyse durch Plasmin: TAFIa Inhibitoren wirken profibrinolytisch.

Um die Hämostase im Blut aufrechtzuerhalten, haben sich Mechanismen ausgebildet, die zur Blutgerinnung und zur Auflösung von Gerinnseln führen; diese stehen in einem Gleichgewicht. Wenn ein gestörtes Gleichgewicht die Koagulation begünstigt, entsteht Fibrin in größeren Mengen, so dass pathologische Vorgänge der Thrombusbildung zu schweren Krankheitsbildern im Menschen führen können.

Genauso wie eine überschießende Koagulation zu schwerwiegenden thrombotisch bedingten Krankheitsbildern führen kann, besitzt eine antithrombotische Behandlung das Risiko von nicht erwünschten Blutungen durch eine Störung der Bildung eines notwendigen hämostatischen Pfropfs. Die Hemmung von TAFIa verstärkt - ohne die Koagulation und die Plättchenaggregation zu beeinflussen - die endogene Fibrinolyse d.h. das gestörte Gleichgewicht wird zugunsten der Fibrinolyse verschoben. So kann sowohl dem Aufbau eines klinisch relevanten Thrombus entgegengewirkt, als auch die Lyse eines schon bestehenden Gerinnsels verstärkt werden. Andererseits wird der Aufbau eines hämostatischen Pfropfs nicht beeinträchtigt, sodass eine Blutungsdiathese eher nicht zu erwarten ist (Bouma et al., J. Thrombosis and Haemostasis, 1, 2003, Seiten 1566 - 1574).

Inhibitoren von TAFIa sind bereits in den Internationalen Anmeldungen WO03/013526 und WO03/061653 beschrieben worden. Einige Imdidazol-essigsäure-derivate als Inhibitoren für TAFIa wurden von Barrow et al. Beschrieben (J. Med. Chem., 2003, Bd. 46, Seiten 5294-5297).

Die erfindungsgemäßen TAFIa Inhibitoren eignen sich für eine prophylaktische als auch für eine therapeutische Anwendung am Menschen, die an Erkrankungen leiden, die mit Thrombosen, Embolien, Hyperkoagulabilität oder fibrotischen Veränderungen einhergehen. Sie eignen sich sowohl für eine akute als auch für eine Langzeittherapie.

Die Erfindung betrifft daher eine Verbindung der Formel la 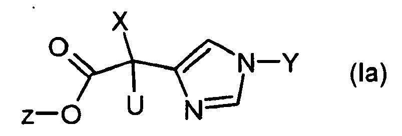 und/oder alle stereoisomeren Formen der Verbindung der Formel Ia und/oder Gemische dieser Formen in jedem Verhältnis, und/oder ein physiologisch verträgliches Salz der Verbindung der Formel Ia, wobei
U für Wasserstoffatom steht,
X für den Rest der Formel II

-(A1)ₘ-A2 (II)

steht, worin
m die ganze Zahl 1 bedeutet,
A1 für -CH₂- steht,
A2 für Aminopyridyl, worin Aminopyridyl unsubstituiert oder unabhängig voneinander ein-, zwei- oder dreifach durch Halogen oder -CH₃
substituiert ist,
Y für den Rest der Formel III

A3-(A4)ₒ-(A5)ₚ (III)

steht, wobei
A3 für -(CH₂)ᵣ-Het steht, worin Het ein Pyrrolidin oder Piperidin ist, welches unsubstituiert oder unabhängig voneinander ein-, zwei- oder dreifach durch R1 substituiert ist,
A4 entfällt,
A5 für a)1) -C(O)-R3,
   a)2) -C(O)-N(R4)-R5,
   a)3) -(SO₂)-R6 oder
   a)4) -C(O)-O-R7 steht,
wobei A5 an das Stickstoffatom von A3 gebunden ist,
p die ganze Zahl 1 und
r die ganze Zahl Null, 1, 2 oder 3 bedeutet,
wobei R1 für
   a) Phenyl, wobei Phenyl unsubstituiert oder ein-, zwei- oder dreifach unabhängig voneinander durch -(C₁-C₄)-Alkyl substituiert ist,
   b) Triazolyl oder Pyridinyl,
   c) -(C₁-C₄)-Alkyl,
   d) -(C₃-C₆)-Cycloalkyl,
   e) -CF₃,
   f) -O-CF3,
   g) Fluor oder
   h) Chlor steht,
wobei R3, R6 und R7 gleich oder verschieden sind unabhängig voneinander für
   a) -(C₁-C₆)-Alkyl, worin Alkyl, unsubstituiert oder unabhängig voneinander ein-, zwei- oder dreifach durch R1 substituiert ist,
   b) Phenyl, worin Phenyl unsubstituiert oder unabhängig voneinander ein-, zwei- oder dreifach durch R1 substituiert ist,
   c) Wasserstoffatom, oder
   d) -(C₃-C₆)-Cycloalkyl, worin Cycloalkyl, unsubstituiert oder unabhängig
voneinander ein-, zwei- oder dreifach durch R1 substituiert ist, stehen, wobei R4 und R5 gleich oder verschieden sind unabhängig voneinander für
   a) -(C₁-C₆)-Alkyl oder -(C₂-C₆)-Alkenyl, worin Alkyl oder Alkenyl, unsubstituiert oder unabhängig voneinander ein-, zwei- oder dreifach durch R1 substituiert ist,
   b) Phenyl, worin Phenyl unsubstituiert oder unabhängig voneinander ein-, zwei- oder dreifach durch R1 substituiert ist,
   c) Wasserstoffatom oder,
   d) -(C₃-C₆)-Cycloalkyl, worin Cycloalkyl, unsubstituiert oder unabhängig voneinander ein-, zwei- oder dreifach durch R1 substituiert ist, stehen, und
Z für
   1) Wasserstoffatom,
   2) -(C₁-C₆)-Alkyl,
   3) -(C₁-C₆)-Alkyl-OH,
   4) -(C₀-C₄)-Alkyl-(C₃-C₆)-Cycloalkyl oder
   5) -(C₁-C₁₀)-Alkyl-O-C(O)-O-(C₃-C₆)-Cycloalkyl steht.

Ein weiterer Gegenstand der Erfindung ist die Verbindung der Formel Ia, wobei U für Wasserstoffatom steht,
X für den Rest der Formel II steht, worin
m die ganze Zahl 1 bedeutet,
A1 für -CH₂- steht,
A2 für den Rest 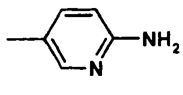 steht, welcher unsubstituiert oder unabhängig
   voneinander ein-, zwei- oder dreifach durch F, CI, Br, J oder -CH₃
   substituiert ist,
Y für den Rest der Formel III steht, wobei
A3 für -(CH₂)ᵣ-Het steht, worin Het ein Pyrrolidin oder Piperidin ist, welches unsubstituiert oder unabhängig voneinander ein-, zwei- oder dreifach durch R1 substituiert ist,
A4 entfällt und
A5 für a)1) -C(O)-R3,
   a)2) -C(O)-N(R4)-R5,
   a)3) -(SO₂)-R6 oder
   a)4) -C(O)-O-R7 steht,
wobei A5 an das Stickstoffatom von A3 gebunden ist,
   p die ganze Zahl 1 und
   r die ganze Zahl Null, 1, 2 oder 3 bedeutet wobei R1 für
      a) Phenyl, wobei Phenyl unsubstituiert oder ein-, zwei- oder dreifach unabhängig voneinander durch -(C₁-C₄)-Alkyl substituiert ist,
      b) Pyridyl oder Tetrazolyl,
      c) -(C₁-C₄)-Alkyl,
      d) -(C₃- C₆)-Cycloalkyl,
      e) -CF₃,
      f) -O-CF3,
      g) Fluor oder
      h) Chlor steht,
wobei R3, R6 und R7 gleich oder verschieden sind unabhängig voneinander für
   a) -(C₁-C₆)-Alkyl, worin Alkyl, unsubstituiert oder unabhängig voneinander ein-, zwei- oder dreifach durch R1 substituiert ist,
   b) Phenyl, worin Phenyl unsubstituiert oder unabhängig voneinander ein-, zwei- oder dreifach durch R1 substituiert ist, oder
   c) -(C₃-C₆)-Cycloalkyl, worin Cycloalkyl, unsubstituiert oder unabhängig
   voneinander ein-, zwei- oder dreifach durch R1 substituiert ist, stehen, wobei R4 und R5 gleich oder verschieden sind unabhängig voneinander für
   a) -(C₁-C₆)-Alkyl oder -(C₂-C₆)-Alkenyl, worin Alkyl oder -(C₂-C₆)-Alkenyl, unsubstituiert oder unabhängig voneinander ein-, zwei- oder dreifach durch R1 substituiert ist,
   b) Phenyl, worin Phenyl unsubstituiert oder unabhängig voneinander ein-, zwei- oder dreifach durch R1 substituiert ist,
   c) Wasserstoffatom oder
   d) -(C₃-C₈)-Cycloalkyl, worin Cycloalkyl, unsubstituiert oder unabhängig voneinander ein-, zwei- oder dreifach durch R1 substituiert ist, stehen,
      und
Z für Wasserstoffatom steht.

Ein weiterer Gegenstand der Erfindung sind Verbindungen der Formel Ia aus der Reihe
3-(6-Amino-pyridin-3-yl)-2-{1-[2-(-diphenylacetyl-piperidin-4-yl)-ethyl]-1H-imidazol-4-yl}-propionsäure, 3-(6-Amino-pyridin-3-yl)-2-{1-[2-(1-benzoyl-piperidin-4-yl)-ethyl]-1H-imidazol-4-yl}-propionsäure, 3-(6-Amino-pyridin-3-yl)-2-[1-(1-benzoyl-piperidin-2-ylmethyl)-1H-imidazol-4-yi]-propionsäure, 3-(6-Amino-pyridin-3-yl)-2-(1-{2-[1-(3-phenyl-propionyl)-piperidin-3-yl]-ethyl}-1H-imidazol-4-yl)-propionsäure, 3-(6-Amino-pyridin-3-yl)-2-[1-(1-diphenylacetyl-piperidin-3-ylmethyl)-1 H-imidazol-4-yl]-propionsäure, 3-(6-Amino-pyridin-3-yl)-2-(1-{2-[1-(3-phenyl-propionyl)-piperidin-4-yl]-ethyl}-1 H- imidazol-4-yl)-propionsäure, 3-(6-Amino-pyridin-3-yl)-2-{1-[2-(1-phenylacetyl-piperidin-3-yl)-ethyl]-1H-imidazol-4-yl}-propionsäure, 3-(6-Amino-pyridin-3-yl)-2-{1-[2-(1-phenylacetyl-piperidin-4-yl)-ethyl]-1H-imidazol-4-yl}-propionsäure, 3-(6-Amino-pyridin-3-yl)-2-{1-[1-(4'-methyl-biphenyl-3-carbonyl)-piperidin-4-ylmethyl]-1H-imidazol-4-yl}-propionsäure oder 3-(6-Amino-pyridin-3-yl)-2-[1-(1-benzoyl-piperidin-4-ylmethyl)-1H-imidazol-4-yl]-propionsäure.

Unter dem Begriff "(C₁-C₆)-Alkyl" oder "(C₁-C₁₀)-Alkyl" werden Kohlenwasserstoffreste verstanden, deren Kohlenstoffkette geradkettig oder verzweigt ist und 1 bis 6 Kohlenstoffatome oder 1 bis 10 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan, Neohexyl, Heptyl, Octanyl, Nonanyl oder Decanyl.

Unter dem Begriff "-(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "C₀-Alkylen" ist eine kovalente Bindung.

Unter dem Begriff "(C₃-C₈)-Cycloalkyl" werden Reste verstanden wie Verbindungen, die sich von 3- bis 8-gliedrige Monocyclen wie Cyclopropyl, Cyclobutyl, Cyclopentyl, Cyclohexyl, Cycloheptyl oder Cyclooctanyl herleiten.

Die erfindungsgemäßen Verbindungen können nach wohlbekannten Verfahren oder nach hier beschriebenen Verfahren hergestellt werden.

Funktionelle Gruppen der verwendeten Intermediate, beispielsweise Amino- oder Carboxylgruppen können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityloder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Green, T.W., Wutz, P.G.M., Protective Groups in Organic Synthesis (1991), 2nd Ed., Wiley-Interscience, oder Kocienski, P., Protecting Groups (1994), Thieme). Der Begriff Schutzgruppe kann auch entsprechende polymergebundene Schutzgruppen umfassen. Solcherart maskierte Verbindungen gemäß Formel (Ia), in der die funktionellen Gruppen des Restes X gegebenenfalls ebenfalls maskiert sein können, können, obwohl gegebenenfalls selber pharmakologisch nicht aktiv, gegebenenfalls nach Administration in Säugern durch Metabolisierung zu den erfindungsgemäßen, pharmakologisch aktiven Verbindungen umgewandelt werden.

Die Erfindung betrifft ferner ein Verfahren zur Herstellung der Verbindung der Formel Ia und/oder einer stereoisomeren Form der Verbindung der Formel Ia und/oder eines physiologisch verträglichen Salzes der Verbindung der Formel Ia, das dadurch gekennzeichnet ist, dass man
a) die Verbindung der Formel Ia nach Schema 1 herstellt, wobei X und Y die jeweils oben angegebenen Bedeutungen haben:

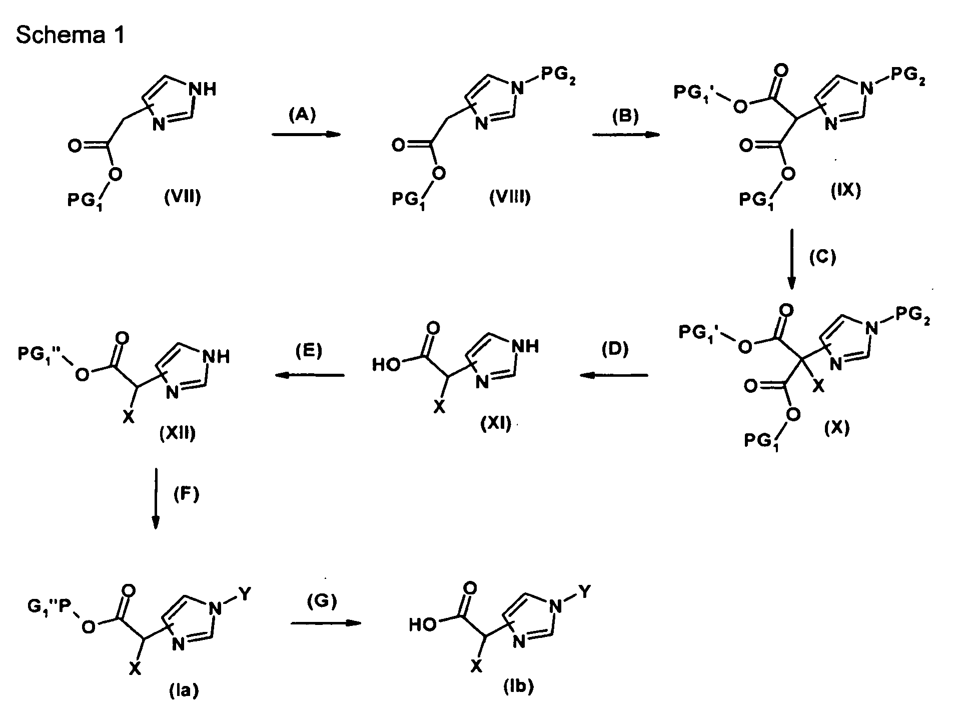

Die Verbindungen gemäß (VII) können nach üblichen Methoden, beispielsweise aus 4-Imidazolessigsäure-Hydrochlorid durch Umsetzung in niederen Alkoholen in Gegenwart von Thionylchlorid erhalten werden, wobei PG1 für eine geeignete Carboxylschutzgruppe steht.

In einem Verfahrensschritt (A) wird nach Standardverfahren eine geeignete Schutzgruppe PG2 eingeführt.

Die resultierenden Verbindungen (VIII) werden im Verfahrensschritt (B) in Gegenwart einer Base in einem inerten Lösungsmittel bei Temperaturen zwischen -90 °C und 50 °C mit einer Verbindung der Formel 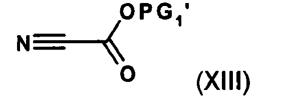 zu den Verbindungen (IX) umgesetzt, wobei PG1' für eine geeignete Carboxylschutzgruppe steht.

Aus den Verbindungen (IX) erhält man im Verfahrensschritt (C) in Gegenwart einer starken Base in einem inerten Lösungsmittel bei Temperaturen zwischen -90 °C und + 50 °C durch Umsetzung mit Verbindungen der Formel

X-LG (XIV)

die Verbindungen (X), wobei in X enthaltene funktionelle Gruppen durch geeignete Schutzgruppen maskiert sein können und LG eine geeignete aktivierende Gruppe wie beispielsweise Chlor, Brom, Iod, Mesylat, Tosylat oder Triflat darstellt.

Im Verfahrensschritt (D) werden die Verbindungen (X) zu den Verbindungen (XI) umgesetzt, indem die Schutzgruppen PG1, PG1' und PG2 sowie gegebenenfalls die in X enthaltene Schutzgruppe nach Standardverfahren entfernt werden und gegebenenfalls unter wäßrig-sauren Bedingungen bei Temperaturen zwischen Raumtemperatur und 100 °C behandelt wird.

Die Verbindungen (XII) können aus den Verbindungen (XI) in einem Schritt (E) erhalten werden, indem unter Standardbedingungen eine geeignete Carboxylschutzgruppe PG1" eingeführt wird.

In einem Schritt (F) können die Verbindungen gemäß (Ia) erhalten werden, indem die Verbindungen (XII) in Gegenwart einer Base bei Temperaturen zwischen -90 °C und +60 °C in einem inerten Lösungsmittel mit Verbindungen der Formel

**Y-LG** (XV)

wobei LG eine geeignete aktivierende Gruppe wie Chlor, Brom, Iod, Mesylat, Tosylat oder Triflat darstellt und Y die oben angegebenen Bedeutungen hat, umgesetzt werden.

Alternativ können die Verbindungen (Ia) erhalten werden, indem die Verbindungen (XII) unter Mitsonobu-Bedingungen mit Verbindungen der Formel

**Y-OH** (XVI),

in denen Y die oben angegebenen Bedeutungen hat, umgesetzt werden.

Alternativ können die Verbindungen (Ia) erhalten werden, indem die Verbindungen (XII) in Gegenwart einer Base bei Temperaturen zwischen -90 °C und +60 °C in einem inerten Lösungsmittel mit sechsgliedrigen 2-Fluronitroaromaten oder sechsgliedrigen 4-Fluronitroaromaten umgesetzt werden. Anschließend wird die Nitrogruppe nach Standardverfahren, z. B. bei Raumtemperatur in niederen Alkoholen mit Wasserstoff in Gegenwart eines Übergangsmetallkatalysators oder in inerten Lösungsmitteln in Gegenwart von Zinn(II)chlorid-Dihydrat zur Aminogruppe reduziert und nach Standardverfahren acyliert.

Die Verbindungen gemäß (Ib) werden im Schritt (G) erhalten, in dem die Schutzgruppe PG1" und gegebenenfalls die in X enthaltene Schutzgruppe unter Standardbedingungen entfernt wird.

Die Verbindungen (XIII), (XIV), (XV) und (XVI) sind kommerziell erhältlich, literaturbekannt oder können nach literaturbekannten Verfahren hergestellt werden.

Die Umsetzungen können bei normalem, erhöhtem oder bei erniedrigtem Druck durchgeführt werden. Im Allgemeinen arbeitet man bei Normaldruck.

Als Lösungsmittel für die Verfahrensschritte (B), (C) und (F) eignen sich inerte organische Lösungsmittel. Hierzu gehören beispielsweise Ether wie Dioxan, THF oder 1,2-Dimethoxyethan, Kohlenwasserstoffe wie Cyclohexan, Benzol, Toluol oder Xylol, Nitroaromaten wie Nitrobenzol, Carbonsäureamide wie Dimethylformamid oder Dimethylacetamid, Alkylsulfoxide wie Dimethylsulfoxid, aliphatische Nitrile wie Acetonitril, oder andere Lösungsmittel wie N-Methylpyrrolidinon. Ebenso ist es möglich, Gemische der genannten Lösungsmittel anzuwenden.

Als Basen für die die Verfahrensschritte (B), (C) und (F) eignen sich die üblichen anorganischen und organischen Basen. Hierzu gehören bevorzugt Alkali- und Erdalkalicarbonate wie Natrium-, Kalium oder Calciumcarbonat, Alkalihydride wie Natriumhydrid, Amide wie Lithium-bis(trimethylsilyl)amid oder Lithium-diisopropylamid, organische Amine wie Pyridin, 4-N,N-Dimethylaminopyridin, Triethylamin, Ethyldiisopropylamin, N-Methylmorpholin, N-Methylpiperidin, 1,5-Diazabicyclo(4.3.0)non-5-en (DBN) oder 1,8-Diazabicyclo(5.4.0)undec-7-en (DBU), oder metallorganische Verbindungen wie Butyllithium oder Phenyllithium. Besonders bevorzugt sind Natriumhydrid, Lithium-bis(trimethylsilyl)amid und Triethylamin.

Unter Mitsonobu-Bedingungen ist im Allgemeinen die Verwendung von inerten Lösungsmitteln in Gegenwart eines Azodicarboxylats, gegebenenfalls in Gegenwart eines Zusatzreagenzes, bevorzugt in einem Temperaturbereich von 0 °C bis Raumtemperatur bei Normaldruck gemeint. Inerte Lösungsmittel sind beispielsweise Halogenkohlenwasserstoffe wie Methylenchlorid, Ether wie Dioxan, THF oder 1,2-Dimethoxyethan, Kohlenwasserstoffe wie Benzol, Toluol oder Xylol, Nitroaromaten wie Nitrobenzol, Carbonsäureamide wie Dimethylformamid oder Dimethylacetamid, Alkylsulfoxide wie Dimethylsulfoxid, aliphatische Nitrile wie Acetonitril, Ester wie Benzoesäureethylester oder andere Lösungsmittel wie N-Methylpyrrolidinon. Ebenso ist es möglich, Gemische der genannten Lösungsmittel anzuwenden.

Übliche Zusatzreagenzien für die Mitsonobu-Reaktion sind beispielweise Triphenylphosphin, Diphenyl-(2-pyridyl)-phosphin oder (4-Dimethylaminophenyl)-diphenylphosphin.

Azodicarboxylate sind beispielsweise Diethylazodicarboxylat, Dimethylazodicarboxylat, Diisopropylazodicarboxylat oder Di-tert-butylazodicarboxylat.

Eine nach Schema 1 hergestellte Verbindung der Formel Ia, oder eine geeignete Vorstufe der Formel Ia, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen

Diastereomeren, und Abspaltung der chiralen Hilfsgruppen in die reinen Enantiomeren auftrennt (Verfahren b), oder
die nach Schema 1 hergestellte Verbindung der Formel Ia entweder in freier Form isoliert oder im Falle des Vorliegens von sauren oder basischen Gruppen in physiologisch verträgliche Salze umwandelt (Verfahren c).

Im Verfahrensschritt b) wird die Verbindung der Formel Ia, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel Ia zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel Ia, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können ggf. auch andere oder vereinfachte Verfahren zur Aufreinigung der Endprodukte eingesetzt werden. Diese Ausgangsprodukte wurden zuvor nach Literatur-bekannten Verfahren enantiomerenoder diastereomerenrein hergestellt. Das kann insbesondere bedeuten, daß in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel Ia können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel Ia, einschließlich deren stereoisomeren Formen, gemäß Verfahrensschritt c) erfolgt in an sich bekannter Weise. Die Verbindungen der Formel Ia bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel Ia basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel Ia und/oder eines physiologisch verträglichen Salzes der Verbindung der Formel Ia und/oder eine gegebenenfalls stereoisomere Form der Verbindung der Formel Ia, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, die durch eine Hemmung von TAFIa behandelbar sind. So eignen sich TAFIa Inhibitoren sowohl für eine prophylaktische als auch für eine therapeutische Anwendung am Menschen. Sie eignen sich sowohl für eine akute Behandlung als auch für eine Langzeittherapie. TAFIa Inhibitoren können eingesetzt werden in Patienten, die an Störungen des Wohlbefindens oder Krankheiten leiden, die mit Thrombosen, Embolien, Hyperkoagulabilität oder fibrotischen Veränderungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können TAFIa Inhibitoren eingesetzt werden bei allen Eingriffen, die zu einem Kontakt des Blutes mit Fremdoberflächen führen wie z. B. bei Dialysepatienten und Patienten mit Verweilkathetern. TAFIa Inhibitoren können eingesetzt werden, um die Thrombosegefahr nach chirurgischen Eingriffen wie bei Knie- und Hüftgelenksoperationen zu reduzieren.

TAFIa Inhibitoren eignen für die Behandlung von Patienten mit disseminierter intravaskulärer Koagulation, Sepsis und anderen intravaskulären Ereignissen, die mit einer Entzündung einhergehen. Weiterhin eignen sich TAFIa Inhibitoren für die Prophylaxe und Behandlung von Patienten mit Atherosklerose, Diabetes und dem metabolischen Syndrom und dessen Folgen. Störungen des hämostatischen Systems (z.B. Fibrinablagerungen) wurden impliziert in Mechanismen, die zu Tumorwachstum und Tumormetastasierung führen; TAFIa Inhibitoren eignen sich zur Verlangsamung oder Verhinderung solcher Prozesse.

Weitere Indikationen für den Einsatz von TAFIa Inhibitoren sind fibrotische Veränderungen der Lunge wie die chronische obstruktive Lungenerkrankung, das adult respiratory distress syndrome (ARDS) und des Auges wie Fibrinablagerungen nach Augenoperationen. TAFIa Inhibitoren eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung mit TAFIa Inhibitoren von Stents und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel Ia mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel Ia enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel Ia, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

TAFIa Inhibitoren können sowohl als Monotherapie als auch in Kombination oder gemeinsam mit allen Antithrombotika (Antikoagulanzien und

Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, RT bedeutet Raumtemperatur (21 °C bis 24 °C). Verwendete Abkürzungen sind entweder erläutert oder entsprechen den üblichen Konventionen.

Wenn nicht anders aufgeführt, wurden die LC/MS-Analysen unter folgenden Bedingungen durchgeführt:
Methode A: Säule: YMC Jsphere 33x2.1 mm, Packungsmaterial 4 µm, Laufmittel:
   CH₃CN + 0.05% Trifluoressigsäure (TFA) : H₂O + 0.05% TFA, Gradient: 5:95 (0 min.) nach 95:5 (3.4 min.), Fluß: 1 mL/min., Temperatur: 30 °C;
Methode B: Säule: YMC Jsphere ODS H80 20x2.1 mm, Packungsmaterial 4 µm, Laufmittel: CH₃CN + 0.05% Trifluoressigsäure (TFA): H₂O + 0.05% TFA, Gradient: 4:96 (0 min.) nach 95:5 (2.0 min.), Fluß: 1 mL/min., Temperatur: 30 °C;
Methode C: Säule: YMC Jsphere 33x2.1 mm, Packungsmaterial 4 µm, Laufmittel: CH₃CN + 0.05% Trifluoressigsäure (TFA) : H₂O + 0.05% TFA, Gradient: 5:95 (0 min.) nach 95:5 (2.5 min.), Fluß: 1.3 mL/min., Temperatur: 30 °C.

Soweit nicht anders angegeben, wurden chromatographische Trennungen an Kieselgel mit Ethylacetat/Heptan-Gemischen als Laufmittel sowie präparative Trennungen an Reversed Phase-(RP)-Kieselgel (HPLC) mit trifluoressigsäurehaltigen Wasser-Acetonitril-Gemischen als Laufmittel durchgeführt.

Das Abdampfen von Lösungsmitteln geschah in der Regel unter vermindertem Druck bei 35 °C bis 45 °C.

### Beispiel 13

3-(6-Amino-pyridin-3-yl)-2-(1-[2-(1-diphenylacetyl-piperidin-4-yl)-ethyl]-1H-imidazol-4-yl}-propionsäure

### Beispiel 13a

1-[4-(2-Hydroxy-ethyl)-piperidin-1-yl]-2,2-diphenyl-ethanon 2-Piperidin-4-yl-ethanol (1,12 g; 8,65 mmol) wurde in Dimethylformamid (DMF, 10 ml) vorge-legt. Dann wurde Hydroxybenzotriazol (HOBT, 1,46 g, 9,51 mmol), 1-Ethyl-3-(3-dimethylamino-propyl)carbodiimid Hydrochlorid (EDC˙HCl, 1,83 g; 9,51 mmol) und Diphenylessigsäure (2,02 g; 9,51 mmol) in dieser Reihenfolge zugegeben. Danach wurde die Reaktionsmischung mit N,N-Diisopropylethylamin (DIEA; 1,67 ml; 9,51 mmol) versetzt und bei RT für 16 h gerührt. Die Reaktionslösung wurde unter vermindertem Druck eingeengt. Der Rückstand wurde in EE aufgenommen und die organische Lösung wurde nacheinander mit gesättigter NaHCO₃-Lösung, 2N HCl und gesättigter NaCl-Lösung gewaschen. Die organische Phase wurde abgetrennt, über Na₂SO₄ getrocknet, filtriert und eingeengt. Das so erhaltene 1-[4-(2-Hydroxy-ethyl)-piperidin-1-yl]-2,2-diphenyl-ethanon (2,39 g; 8,11 mmol) wurde ohne weitere Reinigung weiter umgesetzt.

Rₜ (Methode B) = 1,33 min MS (ES+) = 324 [M+H]⁺

### Beispiel 13b

1-[4-(2-Bromo-ethyl)-piperid in-1-yl]-2,2-diphenyl-ethanon

In Dichlormethan (20 ml) wurde 1-[4-(2-Hydroxy-ethyl)-piperidin-1-yl]-2,2-diphenylethanon (2,39 g; 8,11 mmol) gelöst. Bei Raumtemperatur wurde langsam Phosphortribromid (1,6 ml; 8,92 mmol) zugetropft. Die Reaktionsmischung wurde 4h auf Rückfluß erhitzt. Nach dem Abkühlen wurde mit gesättigter NaHCO₃-Lösung, 2N HCl und gesättigter NaCl-Lösung gewaschen, die organische Phase wurde abgetrennt und über Na₂SO₄ getrocknet. Das Lösemittel wurde unter vermindertem Druck entfernt. Nach Reinigung des Rückstands über eine Kartusche (Silicagel) wurde 1-[4-(2-Bromo-ethyl)-piperidin-1-yl]-2,2-diphenyl-ethanon (0,75 g; 2,13 mmol) erhalten.

Rₜ (Methode B) = 1,79 min MS (ES+) = 386 [M+H]⁺

### Beispiel 13c

3-(6-Amino-pyridin-3-yl)-2-{1-[2-(1-diphenylacetyl-piperidin-4-yl)-ethyl]-1H-imidazol-4-yl}-propionsäure-ethylester

3-(6-Amino-pyridin-3-yl)-2-(1H-imidazol-4-yl)-propionsäureethylester (100 mg; 0,38 mmol) wurde in absolutem DMF (2 ml) gelöst. Es wurde Natriumhydrid (50%; 19 mg; 0,38 mmol) zugegeben und die Reaktionsmischung wurde 1 h bei RT gerührt. Danach wurde 1-[4-(2-Bromo-ethyl)-piperidin-1-yl]-2,2-diphenyl-ethanon (148 mg; 0,38 mmol) in absolutem DMF (1 ml) gelöst zugegeben. Die Reaktionsmischung wurde 1 h bei RT gerührt und dann unter vermindertem Druck eingeengt. Der Rückstand wurde in 1 N HCl aufgenommen und mit Dichlormethan (CH₂Cl₂; 2x) gewaschen. Die wässrige Phase wurde mit 1 N NaOH basisch gestellt und mit CH₂Cl₂ (3x) extrahiert. Die vereinigten organischen Phasen wurden über Na₂SO₄ getrocknet. Nach Entfernen des Lösemittels unter vermindertem Druck wurde 3-(6-Amino-pyridin-3-yl)-2-{1-[2-(1-diphenylacetyl-piperidin-4-yl)-ethyl]-1H-imidazol-4-yl}-propionsäure-ethylester (71 mg; 0,12 mmol) erhalten.

Rₜ (Methode B) = 0,98 min MS (ES+) = 566 [M+H]⁺

### Beispiel 13d

3-(6-Amino-pyridin-3-yl)-2-{1-[2-(1-diphenylacetyl-piperidin-4-yl)-ethyl]-1H-imidazol-4-yl}-propionsäure

In absolutem Ethanol (600 µl) wurde 3-(6-Amino-pyridin-3-yl)-2-{1-[2-(1-diphenylacetyl-piperidin-4-yl)-ethyl]-1H-imidazol-4-yl}-propionsäure-ethylester (71 mg; 0,12 mmol) gelöst. Dazu wurde 1 N NaOH (128 µl; 0,12 mmol) gegeben. Die Reaktionsmischung wurde 16h bei RT gerührt. Danach wurde mit 1 N HCl (128 µl; 0,12 mmol) neutralisiert und unter vermindertem Druck eingeengt. Es wurde in Methanol aufgenommen und über eine C₁₈-Säule gefiltert. Das Methanol wurde entfernt und Acetonitril wurde zugefügt und zweimal dekantiert und der Rückstand danach mit Diethylether verrieben. Der Niederschlag wurde abfiltriert und im Hochvakuum bei 40 °C getrocknet. 3-(6-Amino-pyridin-3-yl)-2-{1-[2-(1-diphenylacetyl-piperidin-4-yl)-ethyl]-1H-imidazol-4-yl}-propionsäure (50 mg; 0,09 mmol) wurde als farbloser Feststoff erhalten. Rₜ (Methode C) = 1,20 min MS (ES+) = 538 [M+H]⁺

¹H-NMR (500 MHz, d⁶-DMSO): 0.90 (m, 1H); 1,02-1,51 (m, 5H); 1,61 (m, 2H); 2,67 (m, 2H); 2,94 (m, 1H); 3,11 (m, 1H); 3,18-3,56 (m, 3H), 3,65 (m, 1H); 3,90 (m, 1H); 4,09 (t; 1H); 5,54 (m; 2H); 6,29 (d,1H); 6,89 (s, 1H); 7,23 (m, 12H); 7,65 (m, 1H)

### Beispiel 14

3-(6-Amino-pyridin-3-yl)-2-{1-[2-(1-benzoyl-piperidin-4-yl)-ethyl]-1H-imidazol-4-yl}-propionsäure

Die Titelverbindung (54 mg; 0,12 mmol) wurde analog zu Beispiel 13 synthetisiert.

Rₜ (Methode C) = 0,88 min MS (ES+) = 448 [M+H]⁺

¹H-NMR (500 MHz, d⁶-DMSO): 1,10 (m, 2H); 1,41 (m, 1H); 1,60 (m, 3H); 1,72 (m, 1H); 2,71 (m; 2H); 2,95 (m, 2H); 3,48 (m, 3H); 3,92 (t, 2H); 4,45 (m, 1H); 5,57 (s; 2H); 6,28 (d, 1H); 6,90 (s, 1H); 7,11 (d, 1H); 7,32 (m, 2H); 7,43 (m, 3H); 7,49 (s, 1H); 7,61 (s, 1H)

### Beispiel 15

3-(6-Amino-pyridin-3-yl)-2-[1-(1-benzoyl-piperidin-2-ylmethyl)-1H-imidazol-4-yl]-propionsäure

Die Titelverbindung (41 mg; 0,09 mmol) wurde analog zu Beispiel 13 synthetisiert.

Rₜ (Methode C) = 0,81 min MS (ES+) = 434 [M+H]⁺

¹H-NMR (500 MHz, d⁶-DMSO): 1,12 (m, 2H); 1,39 (m, 1H); 1,61 (m, 1H); 1,92 (m, 1H); 2,80 (m, 4H); 3,48 (m, 5H); 4,20 (m, 1H); 5,60 (s; 2H); 6,28 (d, 1H); 6,90 (m, 1H); 7,10 (d, 1H); 7,35 (m, 6H); 7,61 (s, 1H)

### Beispiel 16

3-(6-Amino-pyridin-3-yl)-2-(1-{2-[1-(3-phenyl-propionyl)-piperidin-3-yl]-ethyl}-1H-imidazol-4-yl)-propionsäure

Die Titelverbindung (15 mg; 0,03 mmol) wurde analog zu Beispiel 13 synthetisiert.

Rₜ (Methode C) = 1,04 min MS (ES+) = 476 [M+H]⁺

¹H-NMR (500 MHz, d⁶-DMSO): 1,15 (m, 3H); 1,55 (m, 3H); 1,75 (m, 1H); 2,59 (m, 2H); 2, 77 (m, 2H); 2,92 (m, 2H); 3,40 (m, 4H); 3, 65 (m, 1H); 3,89 (t, 2H), 4,15 (m, 1H); 5,55 (s, 2H); 6,28 (d, 1H); 6,89 (m, 1H); 7,13 (m, 2H); 7,22 (m, 4H); 7,45 (s, 1H); 7,63 (m, 1H)

### Beispiel 17

3-(6-Amino-pyridin-3-yl)-2-[1-(1-diphenylacetyl-piperidin-3-ylmethyl)-1H-imidazol-4-yl]-propionsäure

Die Titelverbindung (19 mg; 0,03 mmol) wurde analog zu Beispiel 13 synthetisiert.

Rₜ (Methode C) = 1,13 min MS (ES+) = 524 [M+H]⁺

¹H-NMR (500 MHz, d⁶-DMSO): 0.85-1,75 (m, 5H); 2,55-3,07 (m, 4H); 3,07-3,83 (m, 6H); 4,09 (m; 1H); 5,62 (m; 2H); 6,29 (m,1H); 6,89 (m, 1H); 7,23 (m, 12H); 7,65 (m, 1H)

### Beispiel 18

3-(6-Amino-pyridin-3-yl)-2-(1-{2-[1-(3-phenyl-propionyl)-piperidin-4-yl]-ethyl}-1 H-imidazol-4-yl)-propionsäure

Die Titelverbindung (81 mg; 0,17 mmol) wurde analog zu Beispiel 13 synthetisiert.

Rₜ (Methode C) = 1,00 min MS (ES+) = 476 [M+H]⁺

¹H-NMR (500 MHz, d⁶-OMSO): 0,92 (m, 2H); 1,32 (m, 1H); 1,55 (m, 4H); 2,55 (m, 2H); 2,85 (m, 4H); 3, 40 (m, 4H); 3, 79 (m, 1H); 3,88 (t, 2H), 4,33 (d, 1H); 5,65 (s, 2H); 6,29 (d, 1H); 6,89 (s, 1H); 7,13 (m, 2H); 7,22 (m, 4H); 7,45 (s, 1H); 7,63 (m, 1H)

### Beispiel 19

3-(6-Amino-pyridin-3-yl)-2-{1-[2-(1-phenylacetyl-piperidin-3-yl)-ethyl]-1H-imidazol-4-yl}-propionsäure

Die Titelverbindung (28 mg; 0,06 mmol) wurde analog zu Beispiel 13 synthetisiert.

Rₜ (Methode C) = 0,93 min MS (ES+) = 462 [M+H]⁺

¹H-NMR (500 MHz, d⁶-DMSO): 0,90 (m, 2H); 1,35 (m, 1H); 1,58 (m, 4H); 2,80 (m, 2H); 2,95 (m, 2H); 3, 40 (m, 4H); 3, 69 (s, 1H); 3,85 (t, 2H), 4,33 (d, 1H); 5,60 (s, 2H); 6,29 (d, 1H); 6,90 (s, 1H); 7,10 (dd, 1H); 7,19 (m, 3H); 7,27 (m, 2H); 7,48 (s, 1H); 7,61 (m, 1H)

### Beispiel 20

3-(6-Amino-pyridin-3-yl)-2-{1-[2-(1-phenylacetyl-piperidin-4-yl)-ethyl]-1H-imidazol-4-yl}-propionsäure

Die Titelverbindung (41 mg; 0,08 mmol) wurde analog zu Beispiel 13 synthetisiert.

Rₜ (Methode C) = 0,91 min MS (ES+) = 462 [M+H]⁺

¹H-NMR (500 MHz, d⁶-DMSO): 0,92 (m, 2H); 1,25 (m, 1H); 1,58 (m, 4H); 2,65-4,20 (m, 12H); 5,65 (m, 2H); 6,29 (m, 1H); 6,90 (m, 1H); 7,20 (6H); 7,60 (m, 2H)

### Beispiel 21

3-(6-Amino-pyridin-3-yl)-2-{1-[1-(4'-methyl-biphenyl-3-carbonyl)-piperidin-4-ylmethyl]-1H-imidazol-4-yl}-propionsäure

Die Titelverbindung (33 mg; 0,06 mmol) wurde analog zu Beispiel 13 synthetisiert.

Rₜ (Methode C) = 1,21 min MS (ES+) = 524 [M+H]⁺

¹H-NMR (500 MHz, d⁶-DMSO): 1,12 (m, 2H); 1,45 (m, 2H); 1,95 (m, 1H); 2,36 (s, 3H); 2,80-3,60 (m, 6H); 3,71 (t, 1H); 3,85 (d, 2H); 4,45 (m, 1H); 6,49 (d, 1H); 6,60 (m, 2H); 6,87 (s, 1H); 7,29 (m, 4H); 7,58 (m, 4H); 7,65 (m, 1H); 7,72 (m, 2H)

### Beispiel 22

3-(6-Amino-pyridin-3-yl)-2-[1-(1-benzoyl-piperidin-4-ylmethyl)-1H-imidazol-4-yl]-propionsäure

Die Titelverbindung (46 mg; 0,10 mmol) wurde analog zu Beispiel 13 synthetisiert.

Rₜ (Methode C) = 0,81 min MS (ES+) = 434 [M+H]⁺

¹H-NMR (500 MHz, d⁶-DMSO): 1,10 (m, 2H); 1,45 (m, 2H); 1,95 (m, 1H); 2,80-3,10 (m, 4H); 3,50 (m, 2H) 3,62 (t, 1H); 3,82 (d, 2H); 4,48 (m, 1H); 6,03 (m, 2H); 6,48 (d,1H); 6,90 (d, 1H); 7,19 (d, 1H); 7,35 (m, 2H); 7,42 (m, 3H); 7,55 (s, 1H); 7,61 (s, 1H)

### Pharmakologische Beispiele

Die hergestellten Substanzen wurden mit dem Actichrome Plasma TAFI Activity Kit der Firma American Diagnostica (Pr.Nr. 874) auf Inhibition von TAFIa geprüft. Dabei wurden zu 1 µl 5 mM DMSO-Lösung der Substanz 29 µL Testpuffer (20mM Hepes, 150mM NaCl, pH 7,4) und 10 µL TAFIa (American Diagnostica Pr.Nr. 874TAFIA; 2.5 /ml) gegeben und 15 Minuten bei Raumtemperatur in einer 96 half-well Mikrotiterplatte inkubiert. Die Enzymreaktion wurde durch Zugabe von 10 µL TAFIa "Developer" (1:2 mit Wasser vorverdünnt) gestartet. Der Zeitverlauf der Reaktion wurde bei 420 nm in einem Mikrotiterplattenreader (SpectraMax plus 384; Fa. Molecular Devices) über 15 Minuten verfolgt.

Die IC₅₀ wurde aus den gemittelten Werten (Doppelbestimmung) einer Verdünnungsreihe der Substanz mit Hilfe der Software Grafit 4 (Erithacus Software, UK) berechnet.

Tabelle 1 zeigt die Ergebnisse.

**Tabelle 1:**

| Verbindung aus | TAFIa-Enzym-Assay IC₅₀ [□M] |
|---|---|
| Beispiel 13 | 0,012 |
| Beispiel 15 | 0,012 |
| Beispiel 16 | 0,038 |
| Beispiel 17 | 0,011 |
| Beispiel 18 | 0,021 |

## Patentansprüche

1. Verbindung der Formel la 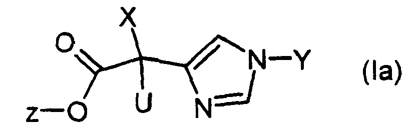 und/oder alle stereoisomeren Formen der Verbindung der Formel Ia und/oder Gemische dieser Formen in jedem Verhältnis, und/oder ein physiologisch verträgliches Salz der Verbindung der Formel Ia, wobei U für Wasserstoffatom steht,
X für den Rest der Formel II
-(A1)ₘ-A2 (II)
steht, worin
m die ganze Zahl 1 bedeutet,
A1 für -CH₂- steht,
A2 für Aminopyridyl, worin Aminopyridyl unsubstituiert oder unabhängig voneinander ein-, zwei- oder dreifach durch Halogen oder -CH₃ substituiert ist,
Y für den Rest der Formel III
A3-(A4)ₒ-(A5)ₚ (III)
steht, wobei
A3 für -(CH₂)ᵣ-Het steht, worin Het ein Pyrrolidin oder Piperidin ist, welches unsubstituiert oder unabhängig voneinander ein-, zwei- oder dreifach durch R1 substituiert ist,
A4 entfällt,
A5 für
a)1) -C(O)-R3,
a)2) -C(O)-N(R4)-R5,
a)3) -(SO₂)-R6 oder
a)4) -C(O)-O-R7 steht,
wobei A5 an das Stickstoffatom von A3 gebunden ist,
p die ganze Zahl 1 und
r die ganze Zahl Null, 1, 2 oder 3 bedeutet,
wobei R1 für
a) Phenyl, wobei Phenyl unsubstituiert oder ein-, zwei- oder dreifach unabhängig voneinander durch -(C₁-C₄)-Alkyl substituiert ist,
b) Triazolyl oder Pyridinyl,
c) -(C₁-C₄)-Alkyl,
d) -(C₃-C₆)-Cycloalkyl,
e) -CF₃,
f) -O-CF3,
g) Fluor oder
h) Chlor steht,
wobei R3, R6 und R7 gleich oder verschieden sind unabhängig voneinander für
a) -(C₁-C₆)-Alkyl, worin Alkyl, unsubstituiert oder unabhängig voneinander ein-, zwei- oder dreifach durch R1 substituiert ist,
b) Phenyl, worin Phenyl unsubstituiert oder unabhängig voneinander ein-, zwei- oder dreifach durch R1 substituiert ist,
c) Wasserstoffatom, oder
d) -(C₃-C₆)-Cycloalkyl, worin Cycloalkyl, unsubstituiert oder unabhängig voneinander ein-, zwei- oder dreifach durch R1 substituiert ist, stehen,
wobei R4 und R5 gleich oder verschieden sind unabhängig voneinander für
a) -(C₁-C₆)-Alkyl oder -(C₂-C₆)-Alkenyl, worin Alkyl oder Alkenyl, unsubstituiert oder unabhängig voneinander ein-, zwei- oder dreifach durch R1 substituiert ist,
b) Phenyl, worin Phenyl unsubstituiert oder unabhängig voneinander ein-, zwei- oder dreifach durch R1 substituiert ist,
c) Wasserstoffatom oder,
d) -(C₃-C₆)-Cycloalkyl, worin Cycloalkyl, unsubstituiert oder unabhängig voneinander ein-, zwei- oder dreifach durch R1 substituiert ist, stehen, und
Z für
1) Wasserstoffatom,
2) -(C₁-C₆)-Alkyl,
3) -(C₁-C₆)-Alkyl-OH,
4) -(C₀-C₄)-Alkyl-(C₃-C₆)-Cycloalkyl oder
5) -(C₁-C₁₀)-Alkyl-O-C(O)-O-(C₃-C₆)-Cycloalkyl steht.

2. Verbindung der Formel Ia gemäß Anspruch 1
und/oder alle stereoisomeren Formen der Verbindung der Formel Ia und/oder Gemische dieser Formen in jedem Verhältnis, und/oder ein physiologisch verträgliches Salz der Verbindung der Formel Ia, wobei
U für Wasserstoffatom steht,
X für den Rest der Formel II steht, worin
m die ganze Zahl 1 bedeutet,
A1 für -CH₂- steht,
A2 für den Rest 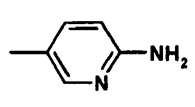 steht, welcher unsubstituiert oder unabhängig voneinander ein-, zwei- oder dreifach durch F, CI, Br, J oder -CH₃ substituiert ist,
Y für den Rest der Formel III steht, wobei
A3 für -(CH₂)ᵣ-Het steht, worin Het ein Pyrrolidin oder Piperidin ist,
welches unsubstituiert oder unabhängig voneinander ein-, zweioder dreifach durch R1 substituiert ist,
A4 entfällt und
A5 für
a)1) -C(O)-R3,
a)2) -C(O)-N(R4)-R5,
a)3) -(SO₂)-R6 oder
a)4) -C(O)-O-R7 steht,
wobei A5 an das Stickstoffatom von A3 gebunden ist,
p die ganze Zahl 1 und
r die ganze Zahl Null, 1, 2 oder 3 bedeutet,
wobei R1 für
a) Phenyl, wobei Phenyl unsubstituiert oder ein-, zwei- oder dreifach unabhängig voneinander durch -(C₁-C₄)-Alkyl substituiert ist,
b) Pyridyl oder Tetrazolyl,
c) -(C₁-C₄)-Alkyl,
d) -(C₃- C₆)-Cycloalkyl,
e) -CF₃,
f) -O-CF3,
g) Fluor oder
h) Chlor steht,
wobei R3, R6 und R7 gleich oder verschieden sind unabhängig voneinander für
a) -(C₁-C₆)-Alkyl, worin Alkyl, unsubstituiert oder unabhängig voneinander ein-, zwei- oder dreifach durch R1 substituiert ist,
b) Phenyl, worin Phenyl unsubstituiert oder unabhängig voneinander ein-, zwei- oder dreifach durch R1 substituiert ist, oder
c) -(C₃-C₆)-Cycloalkyl, worin Cycloalkyl, unsubstituiert oder unabhängig
voneinander ein-, zwei- oder dreifach durch R1 substituiert ist, stehen,
wobei R4 und R5 gleich oder verschieden sind unabhängig voneinander für
a) -(C₁-C₆)-Alkyl oder -(C₂-C₆)-Alkenyl, worin Alkyl oder -(C₂-C₆)-Alkenyl, unsubstituiert oder unabhängig voneinander ein-, zwei- oder dreifach durch R1 substituiert ist,
b) Phenyl, worin Phenyl unsubstituiert oder unabhängig voneinander ein-, zwei- oder dreifach durch R1 substituiert ist,
c) Wasserstoffatom oder
d) -(C₃-C₈)-Cycloalkyl, worin Cycloalkyl, unsubstituiert oder unabhängig voneinander ein-, zwei- oder dreifach durch R1 substituiert ist, stehen, und
Z für Wasserstoffatom steht.

3. Verbindung der Formel Ia gemäß der Ansprüche 1 oder 2, **dadurch gekennzeichnet, dass** es die Verbindung 3-(6-Amino-pyridin-3-yl)-2-{1-[2-(1-diphenylacetyl-piperidin-4-yl)-ethyl]-1H-imidazol-4-yl}-propionsäure, 3-(6-Amino-pyridin-3-yl)-2-{1-[2-(1-benzoyl-piperidin-4-yl)-ethyl]-1H-imidazol-4-yl}-propionsäure,
3-(6-Amino-pyridin-3-yl)-2-[1-(1-benzoyl-piperidin-2-ylmethyl)-1H-imidazol-4-yl]-propionsäure, 3-(6-Amino-pyridin-3-yl)-2-(1-{2-[1-(3-phenyl-propionyl)-piperidin-3-yl]-ethyl}-1H-imidazol-4-yl)-propionsäure, 3-(6-Amino-pyridin-3-yl)-2-[1-(1-diphenylacetyl-piperidin-3-ylmethyl)-1 H-imidazol-4-yl]-propionsäure,
3-(6-Amino-pyridin-3-yl)-2-(1-{2-[1-(3-phenyl-propionyl)-piperidin-4-yl]-ethyl)-1H-imidazol-4-yl)-propionsäure, 3-(6-Amino-pyridin-3-yl)-2-{1-[2-(1-phenylacetyl-piperidin-3-yl)-ethyl]-1H-imidazol-4-yl}-propionsäure, 3-(6-Amino-pyridin-3-yl)-2-{1-[2-(1-phenylacetyl-piperidin-4-yl)-ethyl]-1H-imidazol-4-yl)-propionsäure, 3-(6-Amino-pyridin-3-yl)-2-{1-[1-(4'-methyl-biphenyl-3-carbonyl)-piperidin-4-ylmethyl]-1H-imidazol-4-yl}-propionsäure oder 3-(6-Amino-pyridin-3-yl)-2-[1-(1-benzoyl-piperidin-4-ylmethyl)-1H-imidazol-4-yl]-propionsäure.

4. Verfahren zur Herstellung der Verbindung der Formel Ia gemäß einem oder mehreren der Ansprüche 1 bis 3, **dadurch gekennzeichnet, dass** man
a) eine Verbindung der Formel VII 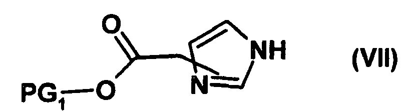 wobei PG1 für eine Carboxylschutzgruppe steht, in eine Verbindung der Formel Ia gemäß Anspruch 1 umwandelt,
b) eine nach Verfahren a) hergestellte Verbindung der Formel Ia, oder eine geeignete Vorstufe der Formel Ia, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel la entweder in freier Form isoliert oder im Falle des Vorliegens von sauren oder basischen Gruppen in physiologisch verträgliche Salze umwandelt.

5. Arzneimittel, **gekennzeichnet durch** einen wirksamen Gehalt an mindestens einer Verbindung der Formel Ia gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung der Verbindung der Formel Ia gemäß einem oder mehreren der Ansprüche 1 bis 3 zur Herstellung eines Arzneimittels zur Prophylaxe und Therapie all solcher Erkrankungen, die die mit Thrombosen, Embolien, Hyperkoagulabilität oder fibrotischen Veränderungen einhergehen.

7. Verwendung gemäß Anspruch 6, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und dessen Folgen, Tumorwachstum und Tumormetastasierung, Störungen des hämostatischen Systems wie Fibrinablagerungen, fibrotische Veränderungen der Lunge wie die chronische obstruktive Lungenerkrankung, das adult respiratory distress syndrome oder Fibrinablagerungen des Auges nach Augenoperationen oder Verhinderung und/oder Behandlung von Narbenbildung.

## Claims

1. A compound of the formula Ia 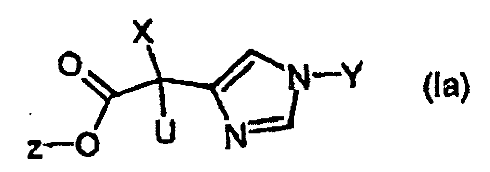
and/or all stereoisomeric forms of the compound of the formula Ia and/or mixtures of these forms in any ratio, and/or a physiologically tolerated salt of the compound of the formula Ia, where
U is hydrogen atom,
X is the radical of the formula II
- (A1)ₘ-A2 (II)
in which
m is the integer 1,
A1 is -CH₂.
A2 is aminopyridyl in which aminopyridyl is unsubstituted or substituted independently of one another once, twice or three times by halogen or CF₃,
Y is the radical of the formula III
A3-(A4)ₒ-(A5)ₚ (III)
where
A3 is -(CH₂)ᵣ-Het in which Het is a pyrrolidine or piperidine which is unsubstituted or substituted independently of one another once, twice or three times by R1,
A4 is absent
A5 is
a)1) -C(O)-R3,
a)2) -C(O)-N(R4)-R5,
a)3) -(SO₂)-R6 or
a)4) -C(O)-O-R7,
where A5 is bonded to the nitrogen atom of A3, p is the integer 1, and
r is the integer zero, 1, 2 or 3,
where R1 is
a) phenyl where phenyl is unsubstituted or substituted independently of one another once, twice or three times by -(C₁-C₄)-alkyl,
b) triazolyl or pyridinyl,
c) - (C₁-C₄) -alkyl,
d) - (C₃-C₆) -cycloalkyl,
e) -CF₃,
f) -O-CF₃,
g) fluorine or
h) chlorine,
where R3, R6 and R7 are identical or different are independently of one another
a) -(C₁-C₆)-alkyl in which alkyl is unsubstituted or substituted independently of one another once, twice or three times by R1,
b) phenyl in which phenyl is unsubstituted or substituted independently of one another once, twice or three times by R1,
c) hydrogen atom, or
d) -(C₃-C₆)-cycloalkyl in which cycloalkyl is unsubstituted or substituted independently of one another once, twice or three times by R1,
where R4 and R5 are identical or different are independently of one another
a) -(C₁-C₆) -alkyl or - (C₂-C₆) -alkenyl, in which alkyl or alkenyl is unsubstituted or substituted independently of one another once, twice or three times by R1,
b) phenyl in which phenyl is unsubstituted or substituted independently of one another once, twice or three times by R1,
c) hydrogen atom, or
d) -(C₃-C₆) -cycloalkyl in which cycloalkyl is unsubstituted or substituted independently of one another once, twice or three times by R1, and
Z is
1) hydrogen atom,
2) -(C₁-C₆)-alkyl,
3) -(C₁-C₆)-alkyl-OH,
4) -(C₀-C₄)-alkyl -(C₃-C₆)-cycloalkyl, or
5) -(C₁-C₁₀)-alkyl-O-C(O)-O-(C₃-C₆)-cycloalkyl.

2. A compound of the formula Ia as claimed in claim 1, and/or all stereoisomeric forms of the compound of the formula Ia and/or mixtures of these forms in any ratio, and/or a physiologically tolerated salt of the compound of the formula Ia, where
U is hydrogen atom,
X is the radical of the formula II in which
m is the integer 1,
A1 is -CH₂-,
A2 is the radical 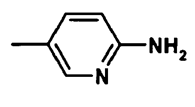 which is unsubstituted or substituted independently of one another once, twice or three times by F, Cl, Br, I or -CH₃,
Y is the radical of the formula III where
A3 is -(CH₂)ᵣ-Het in which Het is a pyrrolidine or piperidine which is unsubstituted or substituted independently of one another once, twice or three times by R1,
A4 is absent and
A5 is
a)1) -C(O)-R3,
a)2) -C(O)-N(R4)-R5,
a)3) -(SO)-R6 or
a)4) -C(O)-O-R7 ,
where A5 is bonded to the nitrogen atom of A3, p is the integer 1, and
r is the integer zero, 1, 2 or 3,
where R1 is
a) phenyl where phenyl is unsubstituted or substituted independently of one another once, twice or three times by -(C₁-C₄)-alkyl,
b) pyridyl or tetrazolyl,
c) -(C₁-C₄) -alkyl,
d) -(C₃-C₆) -cycloalkyl,
e) -CF₃,
f) -O-CF₃,
g) fluorine or
h) chlorine,
where R3, R6 and R7 are identical or different are independently of one another
a) -(C₁-C₆)-alkyl in which alkyl is unsubstituted or substituted independently of one another once, twice or three times by R1,
b) phenyl in which phenyl is unsubstituted or substituted independently of one another once, twice or three times by R1, or
c) -(C₃-C₆)-cycloalkyl, in which cycloalkyl is unsubstituted or substituted independently of one another once, twice or three times by R1,
where R4 and R5 are identical or different are independently of one another
a) -(C₁-C₆) -alkyl or - (C₂-C₆) -alkenyl, in which alkyl or -(C₂-C₆) -alkenyl is unsubstituted or substituted independently of one another once, twice or three times by R1,
b) phenyl in which phenyl is unsubstituted or substituted independently of one another once, twice or three times by R1,
c) hydrogen atom, or
d) -(C₃-C₈) -cycloalkyl in which cycloalkyl is unsubstituted or substituted independently of one another once, twice or three times by R1, and
Z is hydrogen atom.

3. A compound of the formula Ia as claimed in claim 1 or 2, which is the compound
3-(6-Aminopyridin-3-yl)-2-{1-[2-(1-diphenylacetylpiperidin-4-yl)ethyl]-1H-imidazol-4-yl}-propionic acid,
3-(6-Aminopyridin-3-yl)-2-{1-[2-(1-benzoylpiperidin-4-yl)ethyl]-1H-imidazol-4-yl}propionic acid,
3-(6-Aminopyridin-3-yl)-2-[1-(1-benzoylpiperidin-2-ylmethyl)-1H-imidazol-4-yl]propionic acid,
3-(6-Aminopyridin-3-yl)-2-(1-{2-[1-(3-phenylpropionyl)piperidin-3-yl]ethyl}-1H-imidazol-4-yl)propionic acid,
3-(6-Aminopyridin-3-yl)-2-[1-(1-diphenylacetylpiperidin-3-ylmethyl)-1H-imidazol-4-yl]propionic acid,
3-(6-Aminopyridin-3-yl)-2-(1-{2-[1-(3-phenylpropionyl)piperidin-4-yl]ethyl}-1H-imidazol-4-yl)propionic acid,
3-(6-Aminopyridin-3-yl)-2-{1-[2-(1-phenylacetylpiperidin-3-yl)ethyl]-1H-imidazol-4-yl}propionic acid,
3-(6-Aminopyridin-3-yl)-2-{1-[2-(1-phenylacetylpiperidin-4-yl)ethyl]-1H-imidazol-4-yl}propionic acid,
3-(6-Aminopyridin-3-yl)-2-{1-[1-(4'-methylbiphenyl-3-carbonyl)piperidin-4-ylmethyl]-1H-imidazol-4-yl)propionic acid or
3-(6-Aminopyridin-3-yl)-2-[1-(1-benzoylpiperidin-4-ylmethyl)-1H-imidazol-4-yl]propionic acid.

4. A process for preparing the compound of the formula Ia as claimed in one or more of claims 1 to 3, which comprises
a) a compound of the formula VII 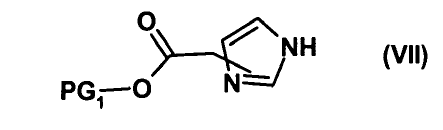 where PG1 is a carboxyl protective group, being converted into a compound of the formula Ia as claimed in claim 1,
b) a compound of the formula Ia which has been prepared by process
a), or a suitable precursor of the formula Ia which occurs owing to its chemical structure in enantiomeric forms being fractionated by salt formation with enantiopure acids or bases, chromatography on chiral stationary phases or derivatization using chiral enantiopure compounds such as amino acids, separation of the diastereomers obtained in this way, and elimination of the chiral auxiliary groups into the pure enantiomers, or
c) the compound of the formula Ia prepared by processes a) or b) being either isolated in free form or, in the case where acidic or basic groups are present, converted into physiologically tolerated salts.

5. A medicament having an effective content of at least one compound of the formula Ia as claimed in one or more of claims 1 to 3 together with a pharmaceutically suitable and physiologically tolerated carrier, additive and/or other active ingredients and excipients.

6. The use of the compound of the formula Ia as claimed in one or more of claims 1 to 3 for producing a medicament for the prophylaxis and therapy of all disorders associated with thromboses, embolisms, hypercoagulability or fibrotic changes.

7. The use as claimed in claim 6, which is applied to myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reduction of the risk of thrombosis following surgical procedures as in knee and hip joint operations, or disseminated intravascular coagulation, sepsis and other intravascular events which are associated with an inflammation, atherosclerosis, diabetes and the metabolic syndrome and its sequelae, tumor growth and tumor metastasis, impairments of the hemostatic system such as fibrin deposits, fibrotic changes of the lung such as chronic obstructive lung disease, adult respiratory distress syndrome or fibrin deposits in the eye after eye operations or prevention and/or treatment of scar formation.

## Revendications

1. Composé de formule Ia 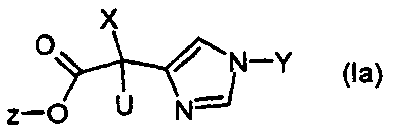
et/ou toutes les formes stéréo-isomères du composé de formule Ia et/ou les mélanges de ces formes, dans tous les rapports, et/ou un sel physiologiquement acceptable du composé de formule Ia, où
U représente un atome d'hydrogène,
X représente le radical de formule II
-(A1)ₘ-A2 (II)
où
m vaut le nombre entier 1,
A1 représente -CH₂-,
A2 représente aminopyridyle, où aminopyridyle
est non substitué ou monosubstitué, disubstitué ou trisubstitué, indépendamment l'un de l'autre, par halogène ou -CH₃,
Y représente le radical de formule III
A3-(A4)ₒ-(A5)ₚ (III)
où
A3 représente - (CH₂)ᵣ-Het, où Het représente
pyrrolidine ou pipéridine, qui est non substitué ou monosubstitué, disubstitué ou trisubstitué, indépendamment l'un de l'autre, par R1, ou
A4 n'existe pas,
A5 représente
a)1) -C(O)-R3,
a)2) -C(O)-N(R4)-R5,
a)3) -(SO₂)-R6 ou
a)4) -C(O)-O-R7,
où A5 est lié à l'atome d'azote de A3,
p vaut le nombre entier 1 et
r vaut le nombre entier zéro, 1, 2 ou 3,
où R1 représente
a) phényle, où phényle est non substitué ou monosubstitué, disubstitué ou trisubstitué, indépendamment l'un de l'autre, par -(C₁-C₄)-alkyle,
b) triazolyle ou pyridinyle,
c) -(C₁-C₄) -alkyle,
d) -(C₃-C₆) -cycloalkyle,
e) -CF₃,
f) -O-CF₃,
g) fluor ou
h) chlore,
où R3, R6 et R7 sont identiques ou différents et représentent, indépendamment l'un de l'autre
a) -(C₁-C₆) -alkyle, où alkyle est non substitué ou monosubstitué, disubstitué ou trisubstitué, indépendamment l'un de l'autre, par R1
b) phényle, où phényle est non substitué ou monosubstitué, disubstitué ou trisubstitué, indépendamment l'un de l'autre, par R1,
c) un atome d'hydrogène ou
d) -(C₃-C₆)-cycloalkyle, où cycloalkyle est non substitué ou monosubstitué, disubstitué ou trisubstitué, indépendamment l'un de l'autre, par R1,
où R4 et R5 sont identiques ou différents et représentent, indépendamment l'un de l'autre
a) -(C₁-C₆) -alkyle ou - (C₂-C₆) -alcényle, où alkyle ou alcényle est non substitué ou monosubstitué, disubstitué ou trisubstitué, indépendamment l'un de l'autre, par R1
b) phényle, où phényle est non substitué ou monosubstitué, disubstitué ou trisubstitué, indépendamment l'un de l'autre, par R1,
c) un atome d'hydrogène ou
d) -(C₃-C₆)-cycloalkyle, où cycloalkyle est non substitué ou monosubstitué, disubstitué ou trisubstitué, indépendamment l'un de l'autre, par R1, et
Z
1) représente un atome d'hydrogène,
2) représente - (C₁-C₆) -alkyle,
3) représente - (C₁-C₆) -alkyl-OH,
4) représente - (C₀-C₄) -alkyl- (C₃-C₆) -cycloalkyle ou
5) représente - (C₁-C₁₀) -alkyl-O-C (O) -O- (C₃-C₆) - cycloalkyle.

2. Composé de formule Ia selon la revendication 1 et/ou toutes les formes stéréo-isomères du composé de formule Ia et/ou les mélanges de ces formes, dans tous les rapports, et/ou un sel physiologiquement acceptable du composé de formule Ia, où
U représente un atome d'hydrogène,
X représente le radical de formule II, où
m vaut le nombre entier 1,
A1 représente -CH₂-,
A2 représente le radical 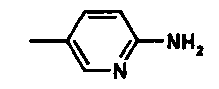
qui est non substitué ou monosubstitué, disubstitué ou trisubstitué, indépendamment l'un de l'autre, par F, Cl, Br, J ou -CH₃,
Y représente le radical de formule III, où
A3 représente -(CH₂)ᵣ-Het, où Het représente
pyrrolidine ou pipéridine,
qui est non substitué ou monosubstitué, disubstitué ou trisubstitué, indépendamment l'un de l'autre, par R1,
A4 n'existe pas et
A5 représente
a)1) - -C(O)-R3,
a)2) -C(O)-N(R4)-R5,
a)3) -(SO₂)-R6 ou
a)4) -C(O)-O-R7,
où A5 est lié à l'atome d'azote de A3,
p vaut le nombre entier 1 et
r vaut le nombre entier zéro, 1, 2 ou 3,
où R1 représente
a) phényle, où phényle est non substitué ou monosubstitué, disubstitué ou trisubstitué, indépendamment l'un de l'autre, par - (C₁-C₄) - alkyle,
b) pyridyle ou tétrazolyle,
c) -(C₁-C₄) -alkyle,
d) -(C₃-C₆) -cycloalkyle,
e) -CF₃,
f) -O-CF₃,
g) fluor ou
h) chlore,
où R3, R6 et R7 sont identiques ou différents et représentent, indépendamment l'un de l'autre
a) -(C₁-C₆) -alkyle, où alkyle est non substitué ou monosubstitué, disubstitué ou trisubstitué, indépendamment l'un de l'autre, par R1
b) phényle, où phényle est non substitué ou monosubstitué, disubstitué ou trisubstitué, indépendamment l'un de l'autre, par R1, ou
c) -(C₃-C₆)-cycloalkyle, où cycloalkyle est non substitué ou monosubstitué, disubstitué ou trisubstitué, indépendamment l'un de l'autre, par R1,
où R4 et R5 sont identiques ou différents et représentent, indépendamment l'un de l'autre
a) - (C₁-C₆) -alkyle ou - (C₂-C₆) -alcényle, où alkyle ou - (C₂-C₆) -alcényle est non substitué ou monosubstitué, disubstitué ou trisubstitué, indépendamment l'un de l'autre, par R1
b) phényle, où phényle est non substitué ou monosubstitué, disubstitué ou trisubstitué, indépendamment l'un de l'autre, par R1,
c) un atome d'hydrogène ou
d) -(C₃-C₈ -cycloalkyle, où cycloalkyle est non substitué ou monosubstitué, disubstitué ou trisubstitué, indépendamment l'un de l'autre, par R1, et
Z représente un atome d'hydrogène.

3. Composé de formule Ia selon les revendications 1 ou 2, **caractérisé en ce qu'**il s'agit du composé acide 3-(6-aminopyridin-3-yl)-2-{1-[2-(1-diphénylacétylpipéridin-4-yl)-éthyl]-1H-imidazol-4-yl}-propionique,
acide 3-(6-aminopyridin-3-yl)-2-{1-[2-(1-benzoylpipéridin-4-yl)-éthyl]-1H-imidazol-4-yl}-propionique,
acide 3-(6-aminopyridin-3-yl)-2-[1-(1-benzoylpipéridin-2-ylméthyl)-1H-imidazol-4-yl]-propionique,
acide 3-(6-aminopyridin-3-yl)-2-(1-{2-[1-(3-phénylpropionyl)-pipéridin-3-yl]-éthyl}-1H-imidazol-4-yl)-propionique,
acide 3-(6-aminopyridin-3-yl)-2-[1-(1-diphénylacétylpipéridin-3-ylméthyl)-1H-imidazol-4-yl]-propionique,
acide 3-(6-aminopyridin-3-yl)-2-(1-{2-[1-(3-phénylpropionyl)-pipéridin-4-yl]-éthyl}-1H-imidazol-4-yl)-propionique,
acide 3-(6-aminopyridin-3-yl)-2-{1-[2-(1-phénylacétylpipéridin-3-yl)-éthyl]-1H-imidazol-4-yl}-propionique,
acide 3-(6-aminopyridin-3-yl)-2-{1-[2-(1-phénylacétylpipéridin-4-yl)-éthyl]-1H-imidazol-4-yl}-propionique,
acide 3-(6-aminopyridin-3-yl)-2-{1-[1-(4'-méthylbiphényl-3-carbonyl)-pipéridin-4-ylméthyl]-1H-imidazol-4-yl}-propionique ou
acide 3-(6-aminopyridin-3-yl)-2-[1-(1-benzoylpipéridin-4-ylméthyl)-1H-imidazol-4-yl]-propionique.

4. Procédé pour la préparation du composé de formule Ia selon l'une ou plusieurs des revendications 1 à 3, **caractérisé en ce qu'**on
a) transforme un composé de formule VII 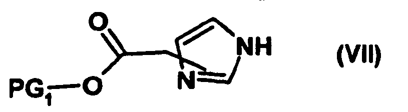 où PG1 représente un groupe de protection de la fonction carboxyle, en un composé de formule Ia selon la revendication 1,
b) sépare un composé de formule Ia, préparé selon le procédé a) ou un précurseur approprié de formule Ia, qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés chiraux sous forme d'énantiomères purs, tels que des aminoacides, séparation des diastéréo-isomères ainsi obtenus et élimination des groupes auxiliaires chiraux, en énantiomères purs, ou
c) soit isole le composé de formule Ia, préparé selon les procédés a) ou b) sous forme libre, soit, dans le cas de la présence de groupes acides ou basiques, convertit en sels physiologiquement acceptables.

5. Médicament, **caractérisé par** une teneur active en au moins un composé de formule Ia selon l'une ou plusieurs des revendications 1 à 3 avec un support, un additif et/ou d'autres substances actives et adjuvants pharmaceutiquement approprié(s) et physiologiquement acceptable (s) .

6. Utilisation du composé de formule Ia selon l'une ou plusieurs des revendications 1 à 3 pour la préparation d'un médicament destiné à la prophylaxie et à la thérapie de toutes les maladies qui vont de pair avec les thromboses, les embolies, l'hypercoagulabilité ou les modifications fibrotiques.

7. Utilisation selon la revendication 6, **caractérisée en ce qu'**il s'agit d'un infarctus du myocarde, d'une angine de poitrine et d'autres formes du syndrome coronaire aigu, d'une attaque, des maladies vasculaires périphériques, de la thrombose veineuse profonde, de l'embolie pulmonaire, de phénomènes emboliques ou thrombotiques provoqués par des arythmies cardiaques, de phénomènes cardiovasculaires tels que la resténose après une revascularisation et une angioplastie et des interventions analogues telles que les implantations d'endoprothèses et les opérations de pontage ou de la réduction du risque d'une thrombose après des interventions chirurgicales, comme lors d'opérations du genou et de la hanche, ou de la coagulation intravasculaire disséminée, la septicémie et d'autres phénomènes intravasculaires, qui vont de pair avec une inflammation, d'athérosclérose, de diabète et du syndrome métabolique et ses conséquences, d'une croissance de tumeur et de la formation de métastases d'une tumeur, de troubles du système hémostatique tels que les dépôts de fibrine, de modifications fibrotiques des poumons, telles que la maladie pulmonaire obstructive chronique, le syndrome de détresse respiratoire adulte (adult respiratory distress syndrome) ou les dépôts de fibrine des yeux après des opérations aux yeux ou de la prophylaxie et/ou du traitement de la formation de cicatrices.
